# EUROPEAN PATENT APPLICATION

(11) **EP 4 147 744 A1**
(43) Date of publication of application: **15.03.2023**
(21) Application number: 21799822.8
(22) Date of filing: 23.03.2021
(51) Int. Cl.: A61M 25/10, A61B 18/24

(54) **CATHETER SYSTEM, CATHETER EQUIPPED WITH CIRCUIT, CIRCUIT, AND FLUID CIRCULATION METHOD**

(30) Priority: 07.05.2020 JP 2020081688
(71) Applicant: Nipro Corporation, Osaka-shi, Osaka 531-8510 (JP)
(72) Inventor: NISHIMURA, Yuki, Osaka-shi, Osaka 531-8510 (JP); SAITO, Yoshikazu, Osaka-shi, Osaka 531-8510 (JP); ITO, Shuntaro, Osaka-shi, Osaka 531-8510 (JP)
(74) Representative: Bertsch, Florian Oliver
(86) International application number: PCT/JP2021/011990
(87) International publication number: WO 2021/225041

(57) **Abstract**

[Problem] To provide an effortless means for deflating a balloon of a catheter.

[Solution] A catheter system 10 includes a catheter 11, a circuit 12 for making fluid flow through the catheter 11, and a drive device 13 having a bidirectionally drivable pump 41 and a controller 44. The catheter 11 includes an inflatable balloon 21, and a first lumen 24 and a second lumen 25 that allow the fluid to flow through the balloon 21. The controller 44 drives the pump 41 in a first direction to make the fluid flow from a buffer tank 31 via a first flow path 32 into the first lumen 24 and to make the fluid flow out from the second lumen 25 via a second flow path 34 to the buffer tank 31, and drives the pump 41 in a second direction opposite to the first direction to make the fluid flow from the first lumen 24 via the first flow path 32 into the buffer tank 31.

## Description

### TECHNICAL FIELD

The present invention relates to a catheter system that makes fluid circulate through a catheter to inflate and deflate a balloon.

### BACKGROUND ART

It is known that when nerves existing in a vicinity of an outer membrane of a renal artery is cauterized, a blood pressure decreases for a long term, and application to a treatment of hypertension is expected. Such a method of cauterizing the nerves in the renal artery is referred to as renal artery sympathetic nerve ablation or renal denervation. In the renal artery sympathetic nerve ablation, for example, there is a method of performing the ablation on the outer membrane that exists at a focal position by guiding a pulse laser to the renal artery using a catheter, condensing the pulse laser on the outer membrane of the renal artery by a condenser lens, and generating a multiphoton absorption at the focal position. A device that makes fluid circulate through a balloon provided to a catheter when performing the ablation is known (Patent Document 1).

### PRIOR ART DOCUMENT

### PATENT DOCUMENT

Patent Document 1: Japanese Patent Application Laid-Open No. 2015-217215

### SUMMARY OF THE INVENTION

### PROBLEMS TO BE SOLVED BY THE INVENTION

The balloon of the catheter is deflated when the catheter is inserted into or removed from a blood vessel, and is inflated when the catheter is placed at a desired position in the blood vessel. A circuit having a flow path for making fluid circulate through the balloon is connected to the catheter. In this circuit, the fluid is made to flow into the balloon to inflate the balloon, the fluid is made to circulate through the inflated balloon, and the fluid is made to flow out from the balloon to deflate the balloon. This requires an operation of switching the flow path using a three-way stopcock connected to the circuit or the like and changing a driving direction of a pump. However, in the circuit to which the three-way stopcock is connected, a risk of an operation mistake with respect to the three-way stopcock is pointed out. Furthermore, it is troublesome to perform an operation of switching the flow path in the circuit in addition to an operation of changing the driving direction of the pump, and the operation mistake may occur due to the necessity of a plurality of operations.

The present invention has been made in view of the above-described circumstances, and an object of the present invention is to provide a means capable of inflating and deflating a balloon of a catheter without operating a circuit.

### MEANS FOR SOLVING THE PROBLEMS

(1) A catheter system according to the present invention includes a catheter, a circuit for making fluid flow through the catheter, and a drive device having a bidirectionally drivable pump and a controller. The catheter includes a shaft, an inflatable balloon provided to the shaft, and a first lumen and a second lumen configured to allow the fluid to flow through the balloon. The circuit includes a buffer tank configured to store the fluid, a first flow path connecting the buffer tank and the first lumen, a second flow path connecting the buffer tank and the second lumen, and a check valve provided on the second flow path and configured to allow the fluid to flow only in one direction in which the fluid flows into the buffer tank. The controller is configured to drive the pump in a first direction to make the fluid flow from the buffer tank via the first flow path into the first lumen and to make the fluid flow out from the second lumen via the second flow path to the buffer tank, and to drive the pump in a second direction opposite to the first direction to make the fluid flow from the first lumen via the first flow path into the buffer tank.
   When the pump is driven in the first direction, the fluid flows through the first flow path and the first lumen into an internal space of the balloon, and the fluid flows out from the internal space of the balloon through the second lumen and the second flow path. At this time, the check valve allows the fluid to pass through the second flow path in a direction in which the fluid flows to the buffer tank. When the pump is driven in the second direction, the fluid flows out from the internal space of the balloon through the first lumen and the first flow path. At this time, the check valve blocks a flow of the fluid in a direction toward the second lumen in the second flow path. Therefore, since the fluid flows out from the internal space of the balloon in a state in which the fluid does not flow into the internal space of the balloon, the balloon is deflated.
(2) Preferably, the circuit further includes a pressure sensor configured to output a detection signal corresponding to a pressure of the fluid in the first flow path. The controller is configured to control driving of the pump in the second direction based on the detection signal obtained from the pressure sensor.
   Based on the detection signal of the pressure sensor, the pump can be driven in the second direction until the balloon is deflated.
(3) Preferably, the catheter includes a laser light irradiation mechanism configured to irradiate laser light outward from an internal space of the balloon.
(4) A catheter with a circuit according to the present invention includes the catheter, and the circuit for making fluid flow through the catheter. The catheter includes a shaft, an inflatable balloon provided to the shaft, and a first lumen and a second lumen configured to allow the fluid to flow through the balloon. The circuit includes a buffer tank configured to store the fluid, a first flow path connecting the buffer tank and the first lumen, a second flow path connecting the buffer tank and the second lumen, and a check valve provided on the second flow path and configured to allow the fluid to flow only in one direction in which the fluid flows into the buffer tank. The fluid is made to flow from the buffer tank via the first flow path into the first lumen, the fluid is made to flow out from the second lumen via the second flow path to the buffer tank, and the fluid is made to flow from the first lumen via the first flow path into the buffer tank.
(5) The present invention relates to a circuit for making fluid flow through a catheter including a first lumen and a second lumen configured to allow the fluid to flow through a balloon. The circuit includes a buffer tank configured to store the fluid, a first flow path connecting the buffer tank and the first lumen, a second flow path connecting the buffer tank and the second lumen, and a check valve provided on the second flow path and configured to allow the fluid to flow only in one direction in which the fluid flows into the buffer tank. The fluid is made to flow from the buffer tank via the first flow path into the first lumen, the fluid is made to flow out from the second lumen via the second flow path to the buffer tank, and the fluid is made to flow from the first lumen via the first flow path into the buffer tank.
(6) The present invention is a fluid circulation method using a catheter system including a catheter having a balloon, a circuit for circulating through the catheter, and a drive device having a bidirectionally drivable pump, wherein the circuit includes a check valve configured to allow fluid to flow only in one direction, and the method includes inflating the balloon by driving the pump to make the fluid flow in the one direction in the circuit, and deflating the balloon by driving the pump to make the fluid flow in a direction opposite to the one direction in the circuit.

### EFFECTS OF THE INVENTION

According to the present invention, the balloon of the catheter can be inflated and deflated without operating the circuit.

### BRIEF DESCRIPTION OF THE DRAWINGS

Fig. 1 is a diagram showing a configuration of a catheter system 10 according to an embodiment of the present invention, including a catheter 11 in a state in which a balloon 21 is in a deflated posture.
Fig. 2 is a cross-sectional diagram of a distal end side of the catheter 11 according to the embodiment of the present invention.
Fig. 3 is a cross-sectional diagram of a vicinity of a connector portion 26 according to the embodiment of the present invention.
Fig. 4 is a flowchart showing a process of inflating the balloon 21 according to the embodiment of the present invention.
Fig. 5 is a flowchart showing a process of deflating the balloon 21 according to the embodiment of the present invention.

### MODES FOR CARRYING OUT THE INVENTION

Hereinafter, a preferred embodiment of the present invention will be described. Note that the present embodiment is merely one embodiment of the present invention, and it is needless to say that the embodiment can be changed without departing from the gist of the present invention.

### [Catheter system 10]

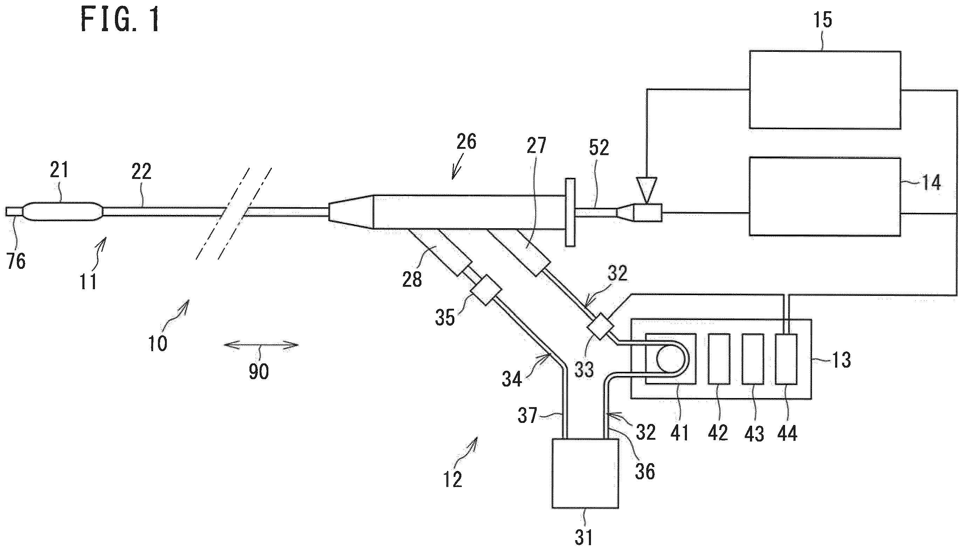

A catheter system 10 is used to perform ablation on nerves of a renal artery, for example. As shown in Fig. 1, the catheter system 10 includes a catheter 11, a circuit 12, a drive device 13, a laser light generation device 14, and a rotation device 15. Note that the catheter system 10 may be used in a known treatment such as a dilatation of a stenosis of a blood vessel.

### [Catheter 11]

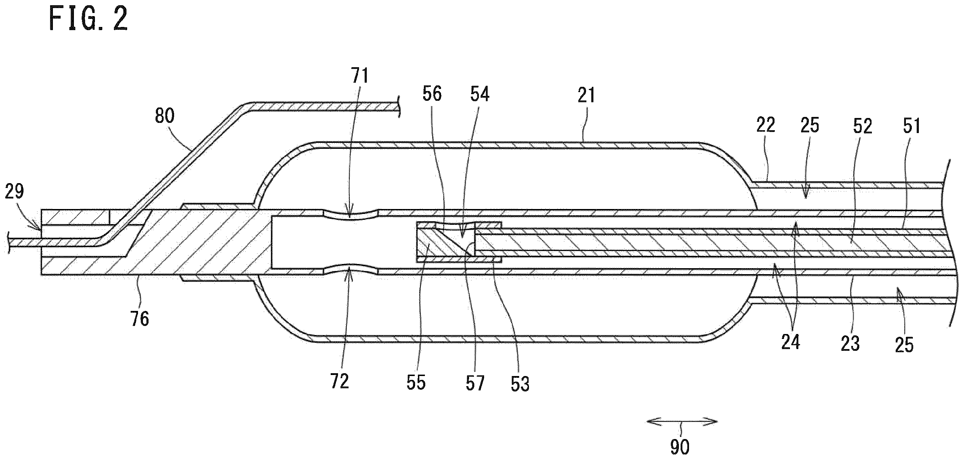

As shown in Figs. 1 and 2, the catheter 11 has a shaft 22 to which a balloon 21 is provided at a distal end side. The shaft 22 is a member elongated in an axial direction 90. The shaft 22 is a tubular body that bows elastically so as to be curved with respect to the axial direction 90. In this specification, a direction in which the shaft 22 in a non-curved state extends is referred to as the axial direction 90.

A fluid tube 23 and a light guide tube 51 are located in an internal space of the shaft 22. The outer diameter and the inner diameter of the shaft 22 need not necessarily be constant with respect to the axial direction 90. The material of the shaft 22 is synthetic resin, stainless steel, or the like, does not necessarily have to be composed of only one kind of material, and may be composed of a plurality of materials. An internal space of the fluid tube 23 is a first lumen 24. Of the internal space of the shaft 22, a space outside the fluid tube 23 is a second lumen 25.

Note that in the present embodiment, a rear side (right side in Fig. 1) with respect to a direction in which the catheter 11 is inserted into a blood vessel is referred to as a proximal end side. A front side (left side in Fig. 1) with respect to the direction in which the catheter 11 is inserted into the blood vessel is referred to as a distal end side.

The balloon 21 is provided on the distal end side of the shaft 22. The balloon 21 is elastically inflated by making the fluid (liquid) flow into an internal space, and is deflated by making the fluid flow out from the internal space. In Fig. 1, the balloon 21 in a deflated state is shown. The fluid flowing through the balloon 21 is not particularly limited, and a mixed solution of saline and a contrast agent is used in the ablation of the renal artery, for example.

The fluid tube 23 penetrates the balloon 21. The fluid tube 23 has openings 71, 72 located in the internal space of the balloon 21. The openings 71, 72 each penetrate a peripheral wall of the fluid tube 23. The first lumen 24 communicates with the internal space of the balloon 21 through the openings 71, 72. The openings 71, 72 are at different positions with respect to a circumferential direction of the axial direction 90.

The second lumen 25, which is the internal space of the shaft 22, is connected to the proximal end side of the balloon 21 and communicates with the internal space of the balloon 21.

The light guide tube 51 is a tube body that can bend elastically so as to be curved with respect to the axial direction 90. A distal end of the light guide tube 51 reaches the internal space of the balloon 21, and a proximal end thereof extends to the outside through the connector portion 26. The light guide tube 51 is movable along the axial direction 90 with respect to the connector portion 26, and is rotatable about the axial direction 90.

An optical fiber 52 is located in an internal space of the light guide tube 51. A distal end surface 57 of the optical fiber 52 is orthogonal to the axis. The optical fiber 52 propagates laser light generated in the laser light generation device 14 and irradiated to a proximal end of the optical fiber 52, to the distal end side thereof. As the optical fiber 52, that having a reflectance for totally reflecting at a wavelength of the laser light is adopted appropriately.

A housing 53 is attached to the distal end of the light guide tube 51. The housing 53 has a tubular shape, and the optical fiber 52 is located in an internal space thereof. The housing 53 has an opening 54 penetrating a peripheral wall. A reflector 55 is located on the distal end side of the opening 54 in the housing 53. In the internal space of the housing 53, the distal end surface 57 of the optical fiber 52 and the reflector 55 are separated, and the opening 54 is located between the distal end surface 57 of the optical fiber 52 and the reflector 55.

The reflector 55 is located in the internal space of the housing 53 so as to face the distal end surface 57 of the optical fiber 52 in the axial direction 90. A reflective surface 56 of the reflector 55, the surface facing the distal end surface 57, is inclined at an angle of 45 degrees with respect to the axial direction 90. The reflective surface 56 is exposed to the outside of the housing 53 through the opening 54 of the housing 53. The reflector 55 is a cylindrical body made of an optical fiber, resin, or the like. A metal layer is laminated on the reflective surface 56 of the reflector 55. For example, the metal layer is formed by plating, sputtering, or the like, with nickel, gold, aluminum, chromium, or the like alone or in a mixed manner, on a surface of the reflector 55.

The optical fiber 52 and the reflector 55 are rotatable about the axis (axial direction 90) integrally with the light guide tube 51 and are slidable in the axial direction 90, in a state in which a positional relationship between the distal end surface 57 and the reflective surface 56, that is, a separation distance and an angle of the reflective surface 56 are maintained. Rotation and slide of the optical fiber 52 and the reflector 55 are controlled by directly or indirectly operating the proximal end side of the light guide tube 51 extending out from the connector portion 26. Specifically, the light guide tube 51 is rotated and slid by applying a driving force from the rotation device 15 to the proximal end side of the light guide tube 51. Note that in the catheter 11, the light guide tube 51, the optical fiber 52, the housing 53, and the reflector 55 constitute a laser light irradiation mechanism that irradiates laser light outward from the internal space of the balloon 21.

As shown in Fig. 1, the connector portion 26 is provided on the proximal end side of the shaft 22. The connector portion 26 is a portion that a practitioner holds when operating the catheter 11. The optical fiber 52 is inserted through the connector portion 26. The connector portion 26 is provided with a first port 27 and a second port 28 apart from an insertion port of the optical fiber 52.

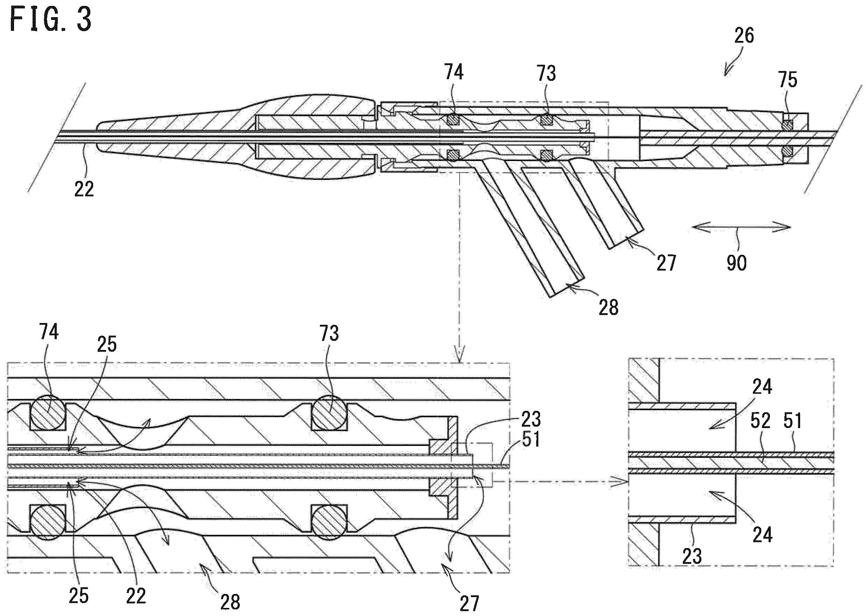

As shown in Fig. 3, the first port 27 is continuous with the first lumen 24. The fluid circulated through the balloon 21 flows into the first lumen 24 through the first port 27. The second port 28 is continuous with the second lumen 25. The fluid circulated through the balloon 21 flows out from the second lumen 25 through the second port 28. Note that the first port 27 and the second port 28 are respectively liquid-tightly separated by O-rings 73, 74 in the connector portion 26. Furthermore, liquid tightness in a periphery of the light guide tube 51 is ensured by an O-ring 75.

As shown in Fig. 2, a distal end tip 76 is provided on the distal end side of the balloon 21. The distal end tip 76 has a guide wire port 29. A guide wire 80 is passed through the guide wire port 29, and the catheter 11 is guided along the guide wire 80 to a desired position in a body of a patient.

### [Circuit 12]

The circuit 12 is used to make the fluid flow through the catheter 11. As shown in Fig. 1, the circuit 12 includes a buffer tank 31, a first flow path 32, a pressure sensor 33, a second flow path 34, and a check valve 35 provided on the second flow path 34.

The buffer tank 31 stores the fluid to be made to flow through the catheter 11 and the circuit 12. The buffer tank 31 is a flexible bag, for example. Although not shown in each drawing, the buffer tank 31 has ports to which the first flow path 32 and the second flow path 34 are connected.

The first flow path 32 is constituted by a synthetic resin tube 36 having flexibility. The first flow path 32 may be constituted by one synthetic resin tube, or may be constituted by joining a plurality of synthetic resin tubes by joints or the like. The first flow path 32 connects the buffer tank 31 and the first port 27 so that the fluid can flow therebetween.

The second flow path 34 is constituted by a synthetic resin tube 37 having flexibility. The second flow path 34 may be constituted by one synthetic resin tube, or may be constituted by joining a plurality of synthetic resin tubes by the joints or the like. The second flow path 34 connects the buffer tank 31 and the second port 28 so that the fluid can flow therebetween. Note that a known joint may be used for connecting each flow path and each port.

The pressure sensor 33 is provided between the first port 27 and a pump 41 in the first flow path 32. The pressure sensor 33 detects a pressure of the fluid in the first flow path 32 and outputs a detection signal corresponding to the detected pressure. As the pressure sensor 33, a diaphragm-type pressure sensor is used, for example. The detection signal of the pressure sensor 33 is output to a controller 44.

The check valve 35 is provided on the second flow path 34. The check valve 35 allows the fluid to flow in one direction and restrains the flow of the fluid in the other direction by a move of a valve provided in a housing, the move caused by a back pressure of the fluid. In the second flow path 34, the check valve 35 allows the fluid to flow, taking a direction in which the fluid flows from the second port 28 to the buffer tank 31 as the one direction. On the other hand, the check valve 35 restrains the flow in a direction in which the fluid flows from the buffer tank 31 to the second port 28, in the second flow path 34.

### [Drive device 13]

The drive device 13 includes the pump 41, a display 42, an input I/F 43, and the controller 44. The pump 41 is preferably a roller pump. The synthetic resin tube 36 constituting the first flow path 32 is set to the pump 41. The pump 41 is bidirectionally drivable.

As the display 42, for example, a liquid crystal display (abbreviation of Liquid Crystal Display), an organic EL display (abbreviation of Organic Electro-Luminescence Display), or the like is adopted.

The input I/F 43 is a user interface that receives an input operation by a user. Specifically, the input I/F 43 is a film-like touch sensor overlapped on the display 42. Alternatively, the input I/F 43 may be a button that the drive device 13 includes. The input I/F 43 receives a user operation and outputs various signals according to the received operation, to the controller 44. An operation signal is inflation of the balloon 21, deflation of the balloon 21, or the like, for example.

The controller 44 has a memory, a CPU, both not shown, and the like. A program is set in advance in the controller 44, and the controller 44 controls operations of the pump 41, operations of the laser light generation device 14, and operations of the rotation device 15, according to the program.

### [Laser light generation device 14]

The laser light generation device 14 is a device in which light of an excitation source is provided to a laser medium, is sent by reflections in an optical resonator, and is output, for example. Laser light output from the laser light generation device 14 is preferably a continuous wave, and the wavelength of the laser light is preferably in a range of 400 to 2,000 nm. The laser light generation device 14 is connected to the proximal end of the optical fiber 52, and the laser light output from the laser light generation device 14 is irradiated to a proximal end surface of the optical fiber 52.

### [Rotation device 15]

The rotation device 15 is a device that applies the driving force for rotating and sliding the proximal end side of the light guide tube 51 with respect to the axial direction 90, and a mechanism in which a motor, a slider, or the like are combined can be adopted. Note that the rotation device 15 is not indispensable, and the light guide tube 51 may be rotated and slid with respect to the axial direction 90 by practitioner's handling of the proximal end side of the light guide tube 51.

### [Fluid circulation method]

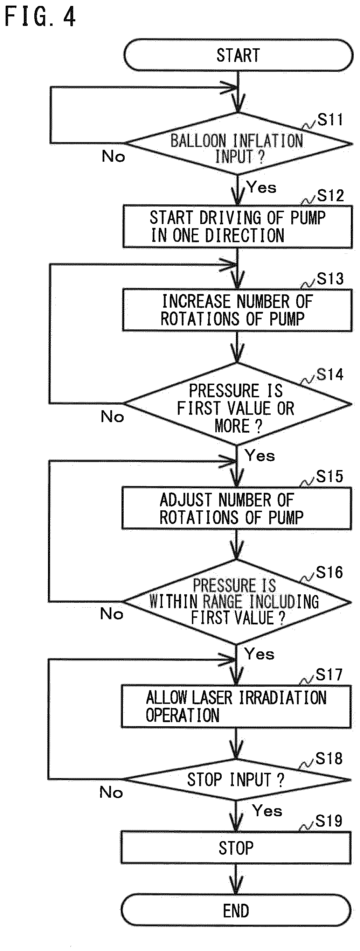

Hereinafter, a fluid circulation method using the catheter system 10 will be described. An inflation of the balloon 21 will be described with reference to Fig. 4.

In the catheter 11, the circuit 12 is connected to each of the ports 27, 28 in advance. In the circuit 12, the synthetic resin tube 36 is set to the pump 41 in advance. The buffer tank 31 is filled with the fluid. In the controller 44, a program suitable for performing the ablation on the renal artery is set in advance. Furthermore, it is assumed that the balloon 21 is in a state in which the balloon 21 is placed at a desired position in the body of the patient.

Under the condition that the controller 44 determines that the operation signal for inflating the balloon 21 is input (S11: Yes), the controller 44 starts driving of the pump 41 in one direction (S12). The pump 41 is driven in the one direction, thereby the fluid flows from the buffer tank 31 into the first flow path 32. The controller 44 obtains the detection signal indicating an internal pressure applied to the synthetic resin tube 36 by the fluid flowing into the first flow path 32, from the pressure sensor 33 at regular time intervals. Note that the controller 44 does not start the driving of the pump 41 until the controller 44 determines that the operation signal for inflating the balloon 21 is input (S11: No). The one direction in which the pump 41 is driven is an example of a first direction.

After starting the driving of the pump 41 (S12), the controller 44 gently increases a unit time of the pump 41, for example, the number of rotations per minute (S13). The number of rotations of the pump 41 is gradually increased, thereby a rapid increase of the internal pressure by the fluid in the circuit 12 is prevented.

The pump 41 continues to rotate in the one direction, thereby the fluid flowing into the first flow path 32 flows from the first port 27 into the first lumen 24. The fluid flowing into the first lumen 24 flows into the internal space of the balloon 21 through the openings 71, 72. The fluid flowing into the balloon 21 flows out from the proximal end side of the balloon 21 to the second lumen 25, and flows out from the second port 28 via the second flow path 34 to the buffer tank 31. The fluid flowing into the buffer tank 31 flows into the first flow path again. In this manner, the fluid circulates through the circuit 12.

The controller 44 determines whether or not the pressure obtained from the pressure sensor 33 becomes equal to or larger than a first value (S14). The controller 44 increases the number of rotations of the pump 41 until the pressure obtained from the pressure sensor 33 becomes equal to or larger than the first value (S14: No). Under the condition that the controller 44 determines that the pressure obtained from the pressure sensor 33 is equal to or larger than the first value (S14: Yes), the controller 44 adjusts the number of rotations of the pump 41 until the pressure obtained from the pressure sensor 33 falls within a range having a predetermined amplitude including the first value (S15 & S16: No).

Since the pressure obtained from the pressure sensor 33 swings to be large or small, for example in accordance with pulses, the pressure has an amplitude regardless of the number of rotations of the pump 41. The amplitude of the pressure obtained from the pressure sensor 33 is said to fall within a range having a predetermined amplitude, when the pressure obtained from the pressure sensor 33 does not deviate from the range having the predetermined amplitude for a predetermined time, such as 5 seconds, 10 seconds, or 30 seconds, for example. For example, adjusting the number of rotations of the pump 41 decelerates the rotations of the pump 41 when the controller 44 determines that the pressure obtained from the pressure sensor 33 is larger than the range having the predetermined amplitude, and accelerates the rotations of the pump 41 when the controller 44 determines that the pressure obtained from the pressure sensor 33 is smaller than the range having the predetermined amplitude. Furthermore, for example, when determining that the pressure obtained from the pressure sensor 33 is continuously larger than the first value for a predetermined time, the controller 44 decreases the number of rotations of the pump 41 per unit time by a predetermined number of rotations, such as half rotation or one rotation, and when determining that the pressure is continuously smaller than the first value for a predetermined time, the controller 44 increases the number of rotations of the pump 41 per unit time by a predetermined number of rotations.

Under the condition that the controller 44 determines that the pressure obtained from the pressure sensor 33 is within the range having the predetermined amplitude including the first value (S16: Yes), the controller 44 allows an irradiation operation of the laser (S17). Note that even after allowing the irradiation operation of the laser, under the condition that the controller 44 determines that the pressure obtained from the pressure sensor 33 deviates from the range having the predetermined amplitude including the first value, the controller 44 adjusts the number of rotations of the pump 41 until the pressure falls within the range having the predetermined amplitude including the first value.

The first value is set to be slightly higher than the pressure of the fluid required for inflating the balloon 21. Furthermore, the first value is set to be lower than the pressure of the fluid when the balloon 21 bursts. When the pressure obtained from the pressure sensor 33 reaches the first value, the balloon 21 is in an inflated state. After the inflation of the balloon 21, the number of rotations of the pump 41 is kept constant without being increased, thereby the balloon 21 is prevented from bursting.

The controller 44 continues to make the pump 41 drive until the operation signal for stopping the driving of the pump 41 is input (S18: No), for example. In response to inputting the operation signal for stopping the driving of the pump 41 (S18: Yes), the controller 44 stops the driving of the pump 41 (S19) . The driving of the pump 41 is stopped, thereby the fluid in the circuit 12 stops circulation in the circuit 12. The circulation of the fluid is stopped, thereby an internal pressure of the internal space of the balloon 21 becomes weak.

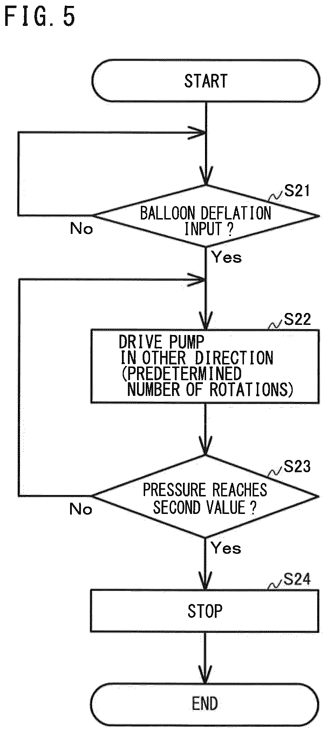

A deflation of the balloon 21 will be described with reference to Fig. 5. When determining that the operation signal for deflating the balloon 21 is input (S21: Yes), the controller 44 drives the pump 41 in the other direction opposite to the one direction (S22). The driving of the pump 41 in the other direction is performed at a predetermined number of rotations at which the balloon 21 is not damaged while the fluid flows through the circuit 12. Note that the controller 44 does not drive the pump 41 in the other direction until determining that the operation signal for deflating the balloon 21 is input (S21: No). The other direction in which the pump 41 is driven is an example of a second direction.

The controller 44 continues to make the pump 41 drive until the pressure obtained from the pressure sensor 33 reaches a second value (S23: No). The pump 41 continues to rotate in the other direction, thereby the fluid in the first flow path 32 flows into the buffer tank 31. At this time, the fluid in the second flow path 34 between the buffer tank 31 and the check valve 35 is restrained from flowing out to a second lumen 25 side from the check valve 35, by the check valve 35. The fluid in the second lumen 25, the internal space of the balloon 21, and the first lumen 24 flows via the first flow path 32 into the buffer tank 31.

Under the condition that the controller 44 determines that the pressure obtained from the pressure sensor 33 reaches the second value (S23: Yes), the controller 44 stops the driving of the pump 41 (S24). The second value is set to a similar level as the pressure when the balloon 21 is in the deflated state. With this, the balloon 21 is deflated.

### [Actions and effects of the present embodiment]

In the present embodiment, when the pump 41 is driven in the first direction, the fluid flows through the first flow path 32 and the first lumen 24 into the internal space of the balloon 21, and the fluid flows out from the internal space of the balloon 21 through the second lumen 25 and the second flow path 34. At this time, the check valve 35 allows the flow of the fluid to flow to the buffer tank 31 in the second flow path 34. When the pump 41 is driven in the second direction, the fluid flows out from the internal space of the balloon 21 through the first lumen 24 and the first flow path 32. At this time, the check valve 35 prevents the fluid from flowing to the second lumen 25 in the second flow path 34. Therefore, since the fluid does not flow into the internal space of the balloon 21, the balloon 21 is deflated.

In the present embodiment, as described above, since the check valve 35 allows or restrains the flow of the fluid in the second flow path 34, the inflation and the deflation of the balloon 21 can be controlled by an operation of changing a driving direction of the pump 41, without performing an operation of switching the second flow path 34 in the circuit 12. In particular, a switching operation of the second flow path 34 in the circuit 12 is not required when the balloon 21 is deflated.

Furthermore, based on the detection signal of the pressure sensor 33, the pump 41 can be driven in the second direction until the balloon 21 is deflated.

### [Modification example]

In the above embodiment, although an example in which the pressure sensor 33 is used is described, the pressure sensor 33 may not be necessarily used. At this time, the controller 44 may control the driving of the pump 41 based on a protocol programmed in advance.

Furthermore, although the check valve 35 is provided on the second flow path 34, the check valve 35 may be provided inside the connector portion 26, not on the second flow path 34. At this time, the check valve 35 is provided in the second port 28 including the proximal end side of the second lumen 25 inside the connector portion 26, for example. Note that when the check valve 35 is provided inside the connector portion 26, the outer shape of the connector portion 26 becomes larger, compared with that in the case where the check valve 35 is provided in the second flow path 34.

Furthermore, the catheter system 10 may not necessarily include the laser irradiation mechanism depending on an application. Furthermore, the catheter system 10 may heat the internal space of the balloon 21 by electrodes for high-frequency conduction or the like, depending on the application.

Furthermore, although the drive device 13 includes the input I/F 43 as the user interface that receives an input by the user, the drive device 13 may not necessarily include the input I/F 43. At this time, the controller 44 may control the driving of the pump 41 based on a protocol programmed in advance.

Furthermore, although the guide wire 80 is passed through the guide wire port 29 of the fluid tube 23, the guide wire port 29 may not necessarily be provided, or a guide wire tube may be provided outside or inside the shaft 22 and the guide wire may be passed through the tube.

### DESCRIPTION OF REFERENCE CHARACTERS

- 10: catheter system
- 11: catheter
- 12: circuit
- 13: drive device
- 14: laser light generation device
- 15: rotation device
- 21: balloon
- 22: shaft
- 24: first lumen
- 25: second lumen
- 31: buffer tank
- 32: first flow path
- 33: pressure sensor
- 34: second flow path
- 35: check valve
- 41: pump
- 44: controller

## Claims

1. A catheter system comprising: a catheter; a circuit for making fluid flow through the catheter; and a drive device having a bidirectionally drivable pump and a controller, wherein
the catheter includes:
a shaft;
an inflatable balloon provided to the shaft; and
a first lumen and a second lumen configured to allow the fluid to flow through the balloon,
the circuit includes:
a buffer tank configured to store the fluid;
a first flow path connecting the buffer tank and the first lumen;
a second flow path connecting the buffer tank and the second lumen; and
a check valve provided on the second flow path and configured to allow the fluid to flow only in one direction in which the fluid flows into the buffer tank, and
the controller is configured:
to drive the pump in a first direction to make the fluid flow from the buffer tank via the first flow path into the first lumen and to make the fluid flow out from the second lumen via the second flow path to the buffer tank, and
to drive the pump in a second direction opposite to the first direction to make the fluid flow from the first lumen via the first flow path into the buffer tank.

2. The catheter system according to claim 1, wherein
the circuit further includes a pressure sensor configured to output a detection signal corresponding to a pressure of the fluid in the first flow path, and
the controller is configured to control driving of the pump in the second direction based on the detection signal obtained from the pressure sensor.

3. The catheter system according to claim 1 or 2, wherein the catheter includes a laser light irradiation mechanism configured to irradiate laser light outward from an internal space of the balloon.

4. A catheter with a circuit comprising: the catheter; and the circuit for making fluid flow through the catheter, wherein
the catheter includes:
a shaft;
an inflatable balloon provided to the shaft; and
a first lumen and a second lumen configured to allow the fluid to flow through the balloon,
the circuit includes:
a buffer tank configured to store the fluid;
a first flow path connecting the buffer tank and the first lumen;
a second flow path connecting the buffer tank and the second lumen; and
a check valve provided on the second flow path and configured to allow the fluid to flow only in one direction in which the fluid flows into the buffer tank, and
the catheter with the circuit is configured so that
the fluid is made to flow from the buffer tank via the first flow path into the first lumen, and the fluid is made to flow out from the second lumen via the second flow path to the buffer tank, and
the fluid is made to flow from the first lumen via the first flow path into the buffer tank.

5. A circuit for making fluid flow through a catheter including a first lumen and a second lumen configured to allow the fluid to flow through a balloon, the circuit comprising:
a buffer tank configured to store the fluid;
a first flow path connecting the buffer tank and the first lumen;
a second flow path connecting the buffer tank and the second lumen; and
a check valve provided on the second flow path and configured to allow the fluid to flow only in one direction in which the fluid flows into the buffer tank, wherein
the circuit is configured so that
the fluid is made to flow from the buffer tank via the first flow path into the first lumen, and the fluid is made to flow out from the second lumen via the second flow path to the buffer tank, and
the fluid is made to flow from the first lumen via the first flow path into the buffer tank.

6. A fluid circulation method using a catheter system comprising: a catheter having a balloon; a circuit for circulating through the catheter; and a drive device having a bidirectionally drivable pump, wherein
the circuit includes a check valve configured to allow fluid to flow only in one direction,
the method comprising:
inflating the balloon by driving the pump to make the fluid flow in the one direction in the circuit; and
deflating the balloon by driving the pump to make the fluid flow in a direction opposite to the one direction in the circuit.
